Europäisches Patentamt

European Patent Office

Office européen des brevets

(11) Publication number: **0 242 553**
**A1**

## EUROPEAN PATENT APPLICATION

(21) Application number: **87103308.0**

(22) Date of filing: **07.03.87**

(51) Int. Cl.⁴: **A61K 33/04** ,
//(A61K33/04,31:415)

(30) Priority: **14.03.86 IT 1974986**

(43) Date of publication of application:
**28.10.87 Bulletin 87/44**

(84) Designated Contracting States:
**AT BE CH DE ES FR GB GR IT LI LU NL SE**

(71) Applicant: **Campo, Giovanni**
**Largo Corsia dei Servi, 11**
**I-20122 Milano(IT)**

(72) Inventor: **Campo, Giovanni**
**Largo Corsia dei Servi, 11**
**I-20122 Milano(IT)**
Inventor: **Di Schiena, Michele G.**
**Via Brunelleschi, 30/C**
**Trezzano Sul Naviglio Milano(IT)**

(74) Representative: **Minoja, Fabrizio**
**Studio Consulenza Brevettuale Via Rossini, 8**
**I-20122 Milano(IT)**

(54) Topical pharmaceutical compositions for use in odontostomatology.

(57) Pharmaceutical compositions for topical use in odontostomatology containing allantoin and sulfur as active principles, show enhanced healing and regenerative effects.

EP 0 242 553 A1

## TOPICAL PHARMACEUTICAL COMPOSITIONS FOR USE IN ODONTOSTOMATOLOGY

The invention refers to pharmaceutical compositions for topical use in odontostomatology, containing as the active principles allantoin and elemental sulfur. The elemental sulfur is known to exist in different forms (for instance: cyclohexasulfur, cycloheptasulfur, $\alpha$, $\beta$ or $\gamma$ sulfur, cubic cyclooctasulfur, cyclodecasulfur, cyclododecasulfur, fibrous sulfur, insoluble sulfur, colloidal sulfur, etc.); it should be understood that the invention comprises all said forms.

The pharmaceutical compositions object of the invention, for their peculiar properties, are particularly useful in the topical therapy of the odontostomatologic diseases such as gingivitis, stomatitis, inflammatory and ulcerative lesions of the oral cavity, parodontopathies, etc.

Both allantoin and sulfur are already used in therapy: allantoin is an effective stimulating agent of the cutaneous tissues regeneration and exhibits re-epithelizing and keratoplastic properties; sulfur exerts a stimulation effect in the tissular metabolic process, has trofic action on the capillary walls and exhibits a repairing and healing activity.

The combination of allantoin and sulfur in the pharmaceutical compositions of the invention surprisingly shows an higher therapeutic effect in comparison with that obtainable with the single components used separately; this may probably be due to a synergistic interction of the two substance.

Said surprising therapeutic characteristic gives to the pharmaceutical compositions of the invention advantageous therapeutic properties, which make them particularly useful in human and veterinary medicine for the treatment of gingival diseases and generally of the oral mucosa, whichever is the etiology causing them and whenever an effective healing, regenerative and lenitive therapy is desired.

In the compositions object of the present invention, the ratio of allantoin and sulfur concentrations is not critical and substantially depends on the considered pharmaceutical form. Generally, allantoin will be present in concentrations from 0.1 to 10%, while the sulfur concentration may be as high as 99.9%.

According to the desired pharmaceutical form, suitable excipients may be used provided that they are compatible; for the powder preparations, for instance, talc, lactose, clay, flavours, dyes, etc. may be used.

For the gel, paste or liquid preparations suitable suspending, aggregating, emulsionating, dispersing, flavouring, colouring agents etc., may be used. Both the different forms and the excipient substances are in any way already known in the considered prior art.

The pharmaceutical compositions of the invention may be added with complementar therapeutic substances such as vitamins (ascorbates, panthotenates, tocopherols, B complex, biotine, Vitamin A), antibiotics, chemotherapics, antiseptic agents, analgesics, antiphlogistic, antimycotic, antiviral, astringent, regulating agents of the oral pH, carriers of organic sulfur.

The following examples further illustrate the invention without limiting it in any way.

### EXAMPLE 1

| | |
|---|---|
| Allantoin | 0.5 g |
| Ventilated sulfur | 99.5 g |

Preparation: the mixture is througly mixed and is then sieved through a fine sieve.

### EXAMPLE 2

| Allantoin | | 5 | g |
| Ventilated sulfur | | 50 | g |
| Excipient: rice starch | q.s. to | 100 | g. |

### EXAMPLE 3

| Allantoin | | 5 | g |
| Ventilated sulfur | | 45 | g |
| Sodium chloride | | 3 | g |
| Excipients: | | | |
| Bolus Alba kaolin | | 30 | g |
| Rice starch | q.s. to | 100 | g. |

### EXAMPLE 4

| Allantoin | | 1.48 | g |
| Ventilated sulfur | | 40 | g |
| Ascorbic acid (Vit. C) | | 1.76 | g |
| Excipient: rice starch | q.s. to | 100 | g. |

### EXAMPLE 5

| Allantoin | | 1.58 | g |
| Ventilated sulfur | | 40 | g |
| Panthotenate calcium | | 4.76 | g |
| Excipient: rice starch | q.s. to | 100 | g. |

## EXAMPLE 6

| Allantoin | | 0.5 | g |
|---|---|---|---|
| Ventilated sulfur | | 20 | g |
| Sodium bicarbonate | | 5 | g |
| Excipients: | | | |
| Hydrated colloidal silica | | 1.5 | g |
| Peppermint alcoholate | | 0.2 | g |
| Rice starch | q.s. to | 100 | g. |

## EXAMPLE 7

| Allantoin | | 5 | g |
|---|---|---|---|
| Ventilated sulfur | | 45 | g |
| Lidocaine hydrochloride | | 1 | g |
| Excipient: rice starch | q.s. to | 100 | g. |

## EXAMPLE 8

| Allantoin | | 0.5 | g |
|---|---|---|---|
| Ventilated sulfur | | 45 | g |
| Cetyltrimethylammonium p-toluensulfonate | | 0.010 | g |
| Excipients: | | | |
| Lactose | | 30 | g |
| Rice starch | q.s. to | 100 | g. |

## EXAMPLE 9

| Allantoin | | 1.58 | g |
|---|---|---|---|
| Ventilated sulfur | | 30 | g |
| Glycirretinic acid | | 4.70 | g |
| Excipient: rice starch | q.s. to | 100 | g. |

## EXAMPLE 10

| Allantoin | | 1.58 | g |
|---|---|---|---|
| Ventilated sulfur | | 30 | g |
| Methionine | | 1.49 | g |
| Excipient: rice starch | q.s. to | 100 | g. |

## EXAMPLE 11

| | |
|---|---|
| Allantoin | 1.58 g |
| Ventilated sulfur | 30 g |
| Clorhexidine | 5.05 g |
| Excipient: rice starch    q.s. to | 100 g. |

## EXAMPLE 12

| | |
|---|---|
| Allantoin | 0.5 g |
| Ventilated sulfur | 3 g |
| Nystatin | 200.000 U. |
| Excipient: rice starch    q.s. to | 5 g. |

## EXAMPLE 13

| | |
|---|---|
| Allantoin | 0.5 g |
| Ventilated sulfur | 30 g |
| Benzidamine hydrochloride | 0.100 g |
| Excipient: rice starch    q.s. to | 100 g. |

## EXAMPLE 14

| | |
|---|---|
| Allantoin | 5 g |
| Ventilated sulfur | 30 g |
| Dexamethasone | 0.05 g |
| Excipient: rice starch    q.s. to | 100 g. |

## EXAMPLE 15

| | |
|---|---|
| Allantoin | 5 g |
| Ventilated sulfur | 30 g |
| Idoxuridine | 1.5 g |
| Neomycin sulfate | 0.6 |
| Excipient: rice starch    q.s. to | 100 g. |

## EXAMPLE 16

| | |
|---|---|
| Allantoin | 5 g |
| Ventilated sulfur | 30 g |
| Zinc citrate | 0.1 g |

| | | | |
|---|---|---|---|
| Excipient: rice starch | q.s. to | 100 | g. |

### EXAMPLE_17

| | | |
|---|---|---|
| Allantoin | 5 | g |
| Ventilated sulfur | 30 | g |
| Aluminium dihydroxyallantoinate | 5 | g |
| Excipient: rice starch    q.s. to | 100 | g. |

### EXAMPLE_18

| | | |
|---|---|---|
| Allantoin | 0.5 | g |
| Ventilated sulfur | 35 | g |
| Excipients: | | |
| Sodium carboxymethylcellulose | 1.5 | g |
| Glycerin | 13 | g |
| Peppermint alcoholate | 0.2 | g |
| Preserved water    q.s. to | 100 | g. |

Preparation: the water is heated to 70° and the sodium carboxymethylcellulose is added in portions. The gel so obtained is added with glycerine, sulfur and allantoin in a blade-mixer.
When the paste is at room temperature, the peppermint alcoholate is added.

### EXAMPLE_19

| | | |
|---|---|---|
| Allantoin | 0.5 | g |
| Ventilated sulfur | 35 | g |
| Sodium chloride | 3 | g |
| Hexetine | 1 | g |
| Excipients: | | |
| Glycerin | 24 | g |
| Colloidal silica | 1.5 | g |
| Sodium carboxymethylcellulose | 1.5 | g |
| Methyl p-hydroxybenzoate | 0.065 | g |

| Propyl p-hydroxybenzoate | | 0.035 g |
| Vanilline | | 0.004 g |
| Calcium and aluminium lake | | 0.015 g |
| Sterile water | q.s. to | 100 g. |

EXAMPLE 20

| Allantoin | | 0.5 g |
| Ventilated sulfur | | 10 g |
| Excipients: | | |
| Liquorice extract | | 1.5 g |
| Peppermint essential oil | | 0.2 g |
| Eucalyptus essential oil | | 0.2 g |
| Sorbitol | | 5 g |
| Glycerin | | 10 g |
| Hydrated colloidal silica | | 2.5 g |
| Sodium carboxymethylcellulose | | 0.5 g |
| Preserved water | q.s. to | 100 g. |

## Claims

1. Oral pharmaceutical compositions for the treatment of gingival diseases or of the oral mucosa consisting of allantoin and sulfur in admixture with suitable inert excipients.

2. Compositions according to claim 1, wherein sulfur is present as $\alpha$, $\beta$ or $\gamma$ sulfur, cyclohexasulfur, cycloheptasulfur, cubic cyclooctasulfur, cyclododecasulfur, fibrous sulfur, insoluble sulfur or colloidal sulfur.

3. Compositions according to claims 1 or 2 wherein allantoin is present in concentrations from O.1 to 1O% and sulfur up to 99.9%.

4. Compositions according to any one of the preceeding claims in liquid forms or in gel, paste or powder form.

5. Compositions according to any one of the previous claim containing other active principle having complementary therapeutic activity.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | UNLISTED DRUGS, vol. 36, no. 11, November 1984, page 210b, Chatham, New Jersey, US; "Acnolisal"<br>* Page 210b, "Acnolisal" * | 1-5 | A 61 K 33/04 //<br>(A 61 K 33/04<br>A 61 K 31:415) |
| X | --- <br>UNLISTED DRUGS, vol. 28, no. 10, October 1976, page 173g, Chatham, New Jersey, US; "Apsor"<br>* Page 1739, "Apsor" * | 1-5 | |
| X | --- <br>UNLISTED DRUGS, vol. 24, no. 8, August 1972, page 120a, Chatham, New Jersey, US; "Actinac"<br>* Page 120a, "Actinac" * | 1-5 | |
| X | --- <br>UNLISTED DRUGS, vol. 20, no. 10, October 1968, page 147b, Chatham, New Jersey, US; "Acne-sol"<br>* Page 147b, "Acne-sol" * | 1-5 | TECHNICAL FIELDS SEARCHED (Int. Cl.4)<br><br>A 61 K |
| X | --- <br>UNLISTED DRUGS, vol. 19, no. 11, November 1967, pages 149f, Chatham, New Jersey, US; "Ellsu"<br>* Page 149f, "Ellsu" * | 1-5 | |
| | ---     -/- | | |

The present search report has been drawn up for all claims

| Place of search | Date of completion of the search | Examiner |
|---|---|---|
| THE HAGUE | 26-06-1987 | PEETERS J.C. |

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| X | ROTE LISTE, 1976, page 337, no. 31331B, Editio Cantor, Aulendorf, DE; "Aknereduct" <br> * Page 337, no. 31331B, "Aknereduct" * <br><br> --- | | |
| X | ROTE LISTE, 1971, pages 40,353,494, Editio Cantor, Aulendorf, DE; "Akneforte Adenylchemie", "Akne-Kaban", "Dexakne Creme", "Fissan-Aknecreme" <br> * Page 40 "Akneforte Adenylchemie", "Akne-Kaban"; page 353 "Dexakne Creme"; page 494 "Fissan-Aknecreme" * <br><br> ----- | 1-5 | |

TECHNICAL FIELDS SEARCHED (Int. Cl.4)

The present search report has been drawn up for all claims

| Place of search | Date of completion of the search | Examiner |
|---|---|---|
| THE HAGUE | 26-06-1987 | PEETERS J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82